# EUROPÄISCHE PATENTSCHRIFT

(11) **EP 2 755 666 B1**
(45) Veröffentlichungstag und Bekanntmachung des Hinweises auf die Patenterteilung: **29.11.2017**
(21) Anmeldenummer: 12772202.3
(22) Anmeldetag: 13.09.2012
(51) Int. Cl.: A61K 35/44, A61K 35/34, A61L 27/22, A61K 35/33, A61P 43/00

(54) **VERFAHREN ZUR HERSTELLUNG EINES BIOLOGISCHEN GEWEBEKONSTRUKTS UND VERWENDUNG SPEZIFISCH GEWONNENER AUTOLOGER ZELLEN UND DESSEN MEDIZINISCHE VERWENDUNG**
METHOD FOR PRODUCING A BIOLOGICAL TISSUE CONSTRUCT AND USE OF SPECIFICALLY OBTAINED AUTOLOGOUS CELLS AND ITS MEDICAL USE
PROCÉDÉ DE FABRICATION D'UN PRODUIT DE SYNTHÈSE TISSULAIRE BIOLOGIQUE ET UTILISATION DE CELLULES AUTOLOGUES OBTENUES DE FAÇON SPÉCIFIQUE ET SON UTILISATION MEDICALE

(30) Priorität: 13.09.2011 DE 102011112955
(43) Veröffentlichungstag der Anmeldung: 23.07.2014
(73) Patentinhaber: Medizinische Hochschule Hannover (MHH), 30625 Hannover (DE)
(72) Erfinder: HAVERICH, Axel, 30177 Hannover (DE); APER, Thomas, 30629 Hannover (DE); WILHELMI, Mathias, 30916 Isernhagen (DE)
(74) Vertreter: Kröncke, Rolf
(86) Internationale Anmeldenummer: PCT/DE2012/000908
(87) Internationale Veröffentlichungsnummer: WO 2013/037349

(56) Entgegenhaltungen:
- WO-A1-2010/123928
- WO-A1-2012/031162
- WO-A2-2007/119240
- WO-A2-2008/077094
- YOUNG P P ET AL: "Biologic Properties of Endothelial Progenitor Cells and Their Potential for Cell Therapy", PROGRESS IN CARDIOVASCULAR DISEASES, SAUNDERS, PHILADELPHIA, PA, US, Bd. 49, Nr. 6, 1. Mai 2007 (2007-05-01), Seiten 421-429, XP026039132, ISSN: 0033-0620, DOI: 10.1016/J.PCAD.2007.02.004 [gefunden am 2007-05-10]
- C.-H. YOON: "Synergistic Neovascularization by Mixed Transplantation of Early Endothelial Progenitor Cells and Late Outgrowth Endothelial Cells: The Role of Angiogenic Cytokines and Matrix Metalloproteinases", CIRCULATION, Bd. 112, Nr. 11, 13. September 2005 (2005-09-13), Seiten 1618-1627, XP055044125, ISSN: 0009-7322, DOI: 10.1161/CIRCULATIONAHA.104.503433
- HUR JIN ET AL: "Characterization of two types of endothelial progenitor cells and their different contributions to neovasculogenesis", ARTERIOSCLEROSIS, THROMBOSIS, AND VASCULAR BIOLOGY, HIGHWIRE PRESS, PHILADELPHIA, PA, US, Bd. 24, Nr. 2, 1. Februar 2004 (2004-02-01), Seiten 288-293, XP009107673, ISSN: 1524-4636, DOI: 10.1161/01.ATV.0000114236.77009.06
- MELERO-MARTIN J M ET AL: "In vivo vasculogenic potential of human blood-derived endothelial progenitor cells", BLOOD, AMERICAN SOCIETY OF HEMATOLOGY, US, Bd. 109, Nr. 11, 1. Juni 2007 (2007-06-01) , Seiten 4761-4768, XP002482185, ISSN: 0006-4971, DOI: 10.1182/BLOOD-2006-12-062471 [gefunden am 2007-02-27]
- HRISTOV M ET AL: "Endothelial progenitor cells in vascular repair and remodeling", PHARMACOLOGICAL RESEARCH, ACADEMIC PRESS, LONDON, GB, Bd. 58, Nr. 2, 1. August 2008 (2008-08-01) , Seiten 148-151, XP025506781, ISSN: 1043-6618, DOI: 10.1016/J.PHRS.2008.07.008 [gefunden am 2008-08-05]
- WU X ET AL: "Tissue-engineered microvessels on three-dimensional biodegradable scaffolds using human endothelial progenitor cells", AMERICAN JOURNAL OF PHYSIOLOGY: HEART AND CIRCULATORY PHYSIOLOGY, AMERICAN PHYSIOLOGICAL SOCIETY, US, Bd. 287, Nr. 2 56-2, 1. August 2004 (2004-08-01), Seiten H480-H487, XP002482180, ISSN: 0363-6135, DOI: 10.1152/AJPHEART.01232.2003

## Beschreibung

Die Erfindung betrifft das Gebiet des Tissue Engineering und der medizinischen Transplantationstechnik. Insbesondere betrifft die Erfindung ein Verfahren zur Herstellung eines artifiziellen biologischen Gewebekonstrukts und die Verwendung spezifisch gewonnener, insbesondere autologer Zellen für dieses Herstellungsverfahren, weitere Besiedlungsverfahren oder die Zelltransplantation.

Seit mehr als 30 Jahren wird auf dem Gebiet des Tissue Engineering daran gearbeitet, körpereigene Materialien, wie Organe, Gefäße oder auch Gewebepatches möglichst gleichwertig durch im Labor hergestellte artifizielle Konstrukte aus biologischen und gegebenenfalls körpereigenen Materialien zu ersetzen. Diese Konstrukte sind als medizinische Transplantate vorgesehen. Im weitesten Sinne zählen hierzu auch Zelltransplantate, beispielsweise zur Rekonstruktion ischämischer Gewebe.

Für Produkte des Tissue Engineering besteht ein sehr hoher Bedarf. Dabei steht die Biokompatibilität im Vordergrund. Das artifizielle Konstrukt soll nach Möglichkeit im Körper so angenommen werden, dass es nicht als fremd erkannt wird und keine pathologischen Reaktionen ausgelöst werden. Auch muss das Konstrukt unmittelbar funktionsfähig sein.

Besondere Probleme bestehen auf dem Arbeitsgebiet noch immer bei der genauen Nachbildung biologischer Gewebe bezüglich der räumlichen Anordnung der darin enthaltenen Zellen, deren Bezug zueinander, einschließlich korrekter Zellkommunikation, Zellfunktion und Gewebeversorgung.

Ein Teilproblem besteht darin, kultivierte Zellen in ein Konstrukt einzubringen, wenn dort noch keine Versorgungsmöglichkeit für die Zellen (Vaskularisierung) besteht.

Die WO 2008/063753 A1 befasst sich mit der Verabreichung von endothelialen Progenitorzellen (EPC) in verletztes Gewebe, um dort eine Neovascularisierung zu induzieren.

Robert E. Verloop et al. beschreiben in "Eur. Cytokine Netw.", Vol. 20, No. 4, 2009, 207 - 209, wie EPCs die Angiogenese in Gewebepräparaten beeinflussen. Untersucht wird hierfür u.a. die Chemokinese und Chemotaxis der EPC auf mit Endothelzellen belegten Substraten.

Aus der WO 2007/119240 A2 ist ein Verfahren zur Knochenregenerierung bekannt. Dabei wurden EPC eingesetzt. Allerdings wird hier nicht die Nutzung von Myoblasten, Myofibroblasten, glatte Muskelzellen oder Vorläufer hiervon und/oder Fibroblasten beschrieben. Die Matrix ist z. B. eine amorphe Kalziumphosphatpaste.

Ein weiteres Teilproblem besteht darin, die Zelladhäsion auf künstlichen Materialien zu gewährleisten, die in einigen Fällen für die mechanische Verstärkung der Konstrukte eingesetzt werden. Selbst wenn es sich dabei um biodegradable Materialien handelt, die im Körper zu körpereigenen Strukturen umgebildet werden (Remodelling), bestehen anfänglich Probleme mit der Zelladhäsion und der Ausbildung konfluenter Oberflächenfilme.

Die Aufgabe der Erfindung besteht darin, bei der Besiedlung von Gewebekonstrukten mit lebenden Zellen oder der Einsiedlung von Zellen in lebendes Gewebe Fortschritte bezüglich des naturgetreuen und biokompatiblen Aufbaus des Besiedlungsresultats zu erzielen und ein verbessertes Verfahren für die Herstellung von Gewebekonstrukten anzugeben.

Diese Aufgabe wird mit den Merkmalen des Verfahrens nach Anspruch 1 und der Verwendung nach Anspruch 9 gelöst. Der Kern der Erfindung ist darin zu sehen, dass eine auf bestimmte Weise erhaltene Zellkultur, die spezifisch gewonnene Zellen, nämlich insbesondere Endothelvorläuferzellen und gegebenenfalls weitere Zellen enthält, für die gewünschten Zwecke, d.h. die Zelltransplantation und die Herstellung bioartifizieller Gewebekonstrukte, sehr vorteilhaft verwendet werden kann. Gemäß einem weiteren Aspekt der Erfindung wird ein besonders vorteilhaftes Herstellungsverfahren bzw. Besiedlungsverfahren für ein Gewebekonstrukt angegeben.

Die hier dargestellten Verfahren zur Herstellung von Gewebekonstrukten für medizinische Zwecke sind grundsätzlich solche, bei welchen lebende Zellen auf eine Matrix aufgesiedelt oder in eine Matrix eingebracht werden, um nach weiteren Behandlungsschritten die gewünschten Gewebekonstrukte zu ergeben.

Unter einer "Matrix" wird hier jedwede Stützstruktur verstanden, auf oder in der Zellen beispielsweise primär durch Adhäsion angesiedelt werden können. Die Matrix steht dabei für die natürliche extrazelluläre Matrix eines Gewebes. Bei vielen Gewebekonstrukttypen bildet die Matrix interstitielles Bindegewebe nach. Die Matrix kann auch ein provisorisches oder permanentes Stützgerüst darstellen, das dem Konstrukt mechanische Festigkeit verleiht. Es kann sich dabei um Metalle oder Kunststoffe handeln. Mit Hilfe eines solchen mechanischen Stützgerüsts wird dann im Allgemeinen die dreidimensionale Grundform des gewünschten Konstrukts nachgeformt. In den meisten Fällen wird heute die Umbildung der Matrix und des Konstrukts insgesamt nach der Transplantation im Körper des Empfängers angestrebt. Es gibt jedoch noch verschiedene Anwendungsgebiete für permanente Stützgerüste, beispielsweise in Form von Stents oder Kunststoffröhrchen.

Beispiele für gängige Matrices im Tissue Engineering sind unter anderem:
- metallische oder keramische Stützgerüste aus Edelstahl, Titan, Keramik, verschiedenen Legierungen, insbesondere im Körper abbaubaren (biodegradablen) Legierungen
- Polymere, wie beispielsweise Polytetrafluorethylen (PTFE), unter anderem verwendet für Gefäßprothesen, die vorzugsweise auf ihrer Oberfläche mit Zellen besiedelt werden, häufig verwendet bei Bypass-Materialien, alternativ Polyterphthalat (z. B. Dacron®), ebenfalls nicht degradabel
- in-vivo abbaubare Kunststoffe, beispielsweise aus Biopolymeren wie Polyzuckern, häufig verwendete Materialien: Polyglycolsäure (Dexon), Polyglactin 910 (Vicryl), Polydioxanon (PDS), Polylactid, Polyepsilonaminocapronsäure (PLC), um nur einige zu nennen. Grundsätzlich sind alle in der Chirurgie als Kleber oder Nahtmaterialien bekannten Materialien geeignet.
- dezelluarisierte biologische Matrices, im Wesentlichen aus Collagen bestehend und bezüglich des vorgesehenen Empfängers (Patientens), soweit möglich autolog, alternativ allogen oder xenogen (vorzugsweise porcin oder bovin). Für die Dezellularisierung geeignete Substrate sind dabei unter anderem Gefäßabschnitte oder Dünndarmmukosa.
- primär azelluläre Proteinpräparationen, z.B. Collagen, Fibrin, Fibrinogen, (kommerziell z.B. Vivostat, Vivostat A/S, DK), Fibronektin, kommerzielle Fibrinkleber (z.B. Tissucol®, Baxter, Heidelberg). Diese Zubereitungen sind vorzugsweise zunächst flüssig, pastös oder gelförmig und können in dieser Form leicht mit den Zellen für die Besiedlung vermischt werden, wobei eine Matrix aus einer solchen Proteinzubereitung im fertigen Konstrukt in der Regel verfestigt bzw. vernetzt vorliegt, um die gewünschte mechanische Stabilität zu liefern. Matrices aus Proteinpräparationen können gegossen, also beispielsweise in einem Spritzgussverfahren verarbeitet werden.

Unter einem "(artifiziellen) Gewebekonstrukt für medizinische Zwecke" wird hier jedes in-vitro gewonnene Gewebe für die verschiedensten Einsatzzwecke verstanden. Das fertige Gewebekonstrukt besteht aus der Matrix, auf- oder eingesiedelten Zellen, gegebenenfalls versehen mit weiteren Hilfsmitteln, wie Wachstumfaktoren, Co-Faktoren, organischchemischen Mitteln zur Stabilisierung, als Spülmedium oder verbliebenes Perfusionsmedium, oder ähnliches, sowie gegebenenfalls Anschlussmitteln für den Anschluss bzw. die Befestigung im Empfängerkörper, beispielsweise Clips. Das Gewebekonstrukt kann beispielsweise ein Gewebepatch, ein Gefäßstück, eine besiedelte Gefäßprothese, ein Bypass-Material oder ein sonstiges Körperorgan oder Organteil sein.

Erfindungsgemäß ist nun vorgesehen, dass die primär auf- oder eingebrachten Zellen EPC (Endothelial Progenitor Cells) enthalten, die nicht mehrfach passagiert wurden und ein Gehalt an EOEC (Early Outgrowth Endothelial Progenitor Cells) und LOEC (Late Outgrowth Endothelial Progenitor Cells) aufweisen. Die letztgenannten Zellen befinden sich vorzugsweise in Mischkultur und weiter vorzugsweise in einer nicht mehrfach passagierten Zellsuspension, d.h. abgelöst und somit vorbereitet für die Verwendung im Besiedlungsverfahren.

Ein großer Vorteil der Erfindung besteht darin, dass die Zellen aus Blut erhältlich sind, und zwar vorzugsweise aus dem Blut des für das Gewebekonstrukt vorgesehenen Empfängers bzw. des Patienten, für den das patientenspezifische Konstrukt gedacht ist. Es handelt sich daher vorzugsweise um autologe Zellen.

Gemäß einer vorteilhaften Verfahrensweise werden die Zellen aus der Monozytenfraktion gewonnen und durch Kultur in spezifischen Kulturmedien, die zellspezifische Supplements enthalten, expandiert und in üblicher Weise inkubiert. Die Kultur erfolgt unter regelmäßigem Wechsel des Kulturmediums über 3 bis 10 Tage. Bis zum Auswachsen koloniebildender Zellen. Erfindungsgemäß ist die gleichzeitige Anwesenheit zweier Zellpopulationen in den Endothelzellkulturen wesentlich. Zunächst treten Zellen auf, die monozytäre Oberflächenmarker CD14 und CD45 exprimieren, jedoch kaum proliferieren. Diese EOEC (Early Outgrowth Endothelial Progenitor Cells) sterben in Kultur innerhalb weniger Wochen ab. Sie werden auch als "colony forming unit-endothelial cells" bezeichnet. Obwohl es sich nach neueren Erkenntnissen wohl nicht um pluripotente Zellen handelt, zeigen diese Zellen ganz wesentliche Eigenschaften, die für den Erfolg des erfindungsgemäßen Besiedlungsverfahrens bzw. für den Erfolg dieser spezifischen Zellen innerhalb von Besiedlungsverfahren und Zelltransplantationen verantwortlich sind.

Zeitlich verzögert zu den EOEC treten sogenannte LOEC (Late Outgrowth Endothelial Progenitor Cells) oder auch "endothelial colony forming cells" auf, die die typische Morphologie von Endothelzellen besitzen und antigene CD34 und CD133 aufweisen. Im Gegensatz zu den EOEC, zeigen die LOEC eine hohe Proliferationsrate. LOEC sind außerdem positiv für das Oberflächenantigen CD31 und die endotheliale NO-Synthase.

Um aus einer begrenzten Menge Blut des Patientens eine genügende Anzahl von Zellen zu erhalten, ist es im Stand der Technik üblich, die Zellen bei der Kultivierung mehrfach zu passagieren und so zu vermehren. Hierfür werden nach Erreichen der Konfluenz in der Erstkultur die Zellen mit Trypsin abgelöst und im Allgemeinen 1:3 passagiert, also von einer auf drei Kulturflaschen aufgeteilt. Dieser Vorgang wird mehrfach wiederholt, um eine ausreichende Zellmenge für eine erfolgreiche Besiedlung auf der Trägerstruktur zu erhalten. Üblich ist die Verwendung der vierten oder fünften Passage.

Erfindungsgemäß werden nun die Zellen aus der Endothelzellenkultur nicht mehrfach passagiert, also maximal einmal passagiert. Verwendet werden die Zellen der ersten Passage oder der Ursprungskultur, d.h. es wird bewusst auf die exponentielle Vermehrung während längerer Kulturzeiten verzichtet und mit einer geringeren Zellzahl gearbeitet, da nur so die für den Besiedlungserfolg wesentlichen EOEC in ausreichender Menge erhalten bleiben.

Alternativ zu der vorstehend beschriebenen Kultivierung können die erforderlichen Zellen beispielsweise aus Vollblut mittels spezifischer Antikörper isoliert werden. Die verwendeten Zellsuspensionen enthalten dann vorzugsweise (typischerweise) Zellen mit den Oberflächenantigenen CD31/CD34 (zirkulierende Endothelzellen entsprechend LOEC), Zellen, die CD14/CD45 exprimieren aber nicht CD31 und CD34 sowie aus Zellen, die kontraktile Filamente wie Alpha-Actin exprimieren (zirkulierende glatte Muskelzellen, Myofibroblasten, Fibroblasten/Fibrozyten). Eine solche Mischkultur findet sich nach Auswachsen der Zellen aus der Monozytenfraktion, bevor die Kulturen passagiert werden. Durch Separation mittels spezifischer Antikörper, die z.B. an magnetische Partikel gebunden sind, können diese Mischkulturen gezielt aus Vollblut isoliert werden.

Überraschender Weise wurde gefunden, dass die Besiedlung der Matrices auch bei anfänglich geringer Zelldichte der für die Besiedlung gewählten Endothelzellen sehr gut möglich ist. So fällt bereits bei der Kultivierung der Zellen auf, dass sich die Zellen in einer Kulturflasche gleichmäßig ausbreiten. Die Proliferation der Zellinseln, in denen es bei zunehmender Zelldichte zum Absterben von Zellen kommt, wie bei reifen aus Aorten isolierten Endothelzellen (aEC), wird nicht beobachtet. Außerdem wird ein beträchtlicher Anteil (nicht abgestorbener) schwimmender Zellen beobachtet, die sich bei Inkontaktbringen mit einer Matrix sehr leicht ansiedeln lassen. Die erfindungsgemäß nicht mehrfach passagierten EPC zeigen eine deutlich verbesserte Adhäsion sowohl in biologischen Matrices, wie azellulären Proteinpräparationen, als auch gegenüber Kunststoffunterlagen, beispielsweise aus PTFE. Die erfindungsgemäßen EPC besitzen demnach eine deutlich höhere Kapazität, auf verschiedenen Oberflächen eine konfluente Schicht auszubilden. Weiterhin zeigt sich eine signifikante höhere NO-Bildungsrate, eine verbesserte Stimulierbarkeit der NO-Synthase und eine bessere Überlebensfähigkeit als bei mehrfach passagierten EPC.

Erfindungsgemäß werden gleichzeitig oder sequentiell außer EPC-Zellen ein oder mehrere Zelltypen ausgewählt aus der Gruppe:
Myoblasten, Myofibroblasten, glatte Muskelzellen (SMC, smooth muscle cells), Vorläuferzellen der glatten Muskelzellen (SPC, smooth muscle progenitor cells), Fibroblasten auf- oder eingebracht. Mit den Zellen können optional zusätzliche Faktoren, beispielsweise Wachstumsfaktoren mit eingebracht werden.

Der Begriff "Fibroblasten" ist dabei im allgemeinsten Sinne zu verstehen. Hierzu werden hier u.a. im Blut zirkulierende Fibroblasten gezählt, die in der Literatur auch als "Fibrozyten" bezeichnet werden.

Es hat sich herausgestellt, dass die Ausbildung eines funktionellen Endothels schneller erfolgt, wenn gleichzeitig Muskelzellen, nämlich Myoblasten, Myofibroblasten, glatte Muskelzellen oder deren Vorläufer in oder auf der Matrix vorhanden sind. Diese Zellen beeinflussen sich untereinander vorteilhaft. Der Auf- und Umbau des Konstrukts wird dadurch beschleunigt.

In Weiterbildung der Erfindung können zusätzlich Fibroblasten auf- oder eingebracht werden. Durch die Fibroblasten wird zusätzliches Kollagen gebildet, die extrazelluläre Matrix des Konstrukts wird ausgehend von der ursprünglich vorgegebenen Matrix weiter stabilisiert, und es wird ein mechanisch besonders stabiles Ergebnis erhalten.

Je nach gewählter Matrix für das Konstrukt wird der Fachmann wählen, ob die Zellen gleichzeitig oder sequentiell, d.h. in bestimmter Folge nacheinander auf die Matrix aufgesiedelt bzw. in ein Matrixmaterial eingebracht werden.

Gemäß einem besonders bevorzugten Ausführungsbeispiel der Erfindung wird als Matrix eine Proteinzubereitung verwendet und die Zellen werden gleichzeitig oder in beliebiger Reihenfolge in diese Zubereitung eingemischt. Im weiteren Verlauf des Besiedlungsverfahrens organisieren sich die Zellen innerhalb der Struktur selbst. Vorzugsweise kann eine Fibrinogenzubereitung verwendet werden, oder eine Zubereitung aus Fibrinopeptiden als Vorstufe zu Fibrinogen.

Weiterhin ist es besonders bevorzugt, dass die bei dem Verfahren eingesetzten Zellen oder wenigstens die bei dem Verfahren eingesetzten EPC aus Blut gewonnen wurden. Hierdurch ist es auf sehr einfache Weise möglich, das Gewebekonstrukt mit autologen Zellen bezüglich des vorgesehenen Empfängers auszustatten. Aus einer einzigen Blutprobe können alle für die Besiedlung gewünschten Zellen isoliert und expandiert werden.

Alternativ können bestimmte Zellen auch aus Fettgewebe gewonnen werden. Dies erweitert die Möglichkeiten zur Gewinnung autologer Zellen. Hierfür wird dem Patienten, der das Gewebekonstrukt erhalten soll, Fettgewebe entnommen, wie dies beispielsweise für Fetttransplantationen bekannt ist. Aus dem Fettgewebe werden mit bekannten Verfahren Fettzellen und andere Zellen gewonnen. Die Zellgewinnung aus Fettgewebe eignet sich insbesondere zur Gewinnung der für das erfindungsgemäße Verfahren optional eingesetzten Fibroblasten.

Die auf die vorstehende Weise gewonnenen Zellen, und zwar erfindungsgemäß wenigstens nicht mehrfach passagierte EPC mit einem Gehalt an EOEC und LOEC, in Verbindung mit Muskelzellen und in besonderen Ausführungsformen mit Fibroblasten, auch zusätzlich zu anderen Muskelzellen, werden nun vorzugsweise auf eine Prothese oder Matrix aus wenigstens einem Material der Gruppe:
in vivo nicht abbaubares biokompatibles Material, in vivo abbaubares biodegradables synthetisches Material; in vivo abbaubares biodegradables biologisches Material, insbesondere eine primär zellfreie Proteinzubereitung,
aufgesiedelt oder in ein solches Material eingebracht.

Dabei ist es besonders bevorzugt, dass als Matrix eine primär azelluläre Proteinzubereitung verwendet wird, und zwar insbesondere eine Fibrinogenzubereitung. Die Fibrinogenzubereitung kann in besonders vorteilhafter Weise ebenfalls aus Blut gewonnen werden, und zwar in besonders bevorzugter Ausführungsform des Verfahrens aus derselben Blutprobe wie die für die Besiedlung verwendeten Zellen.
Für die Herstellung eines Fibrinogen- oder Fibrinpräparats aus Blut sind verschiedene Verfahren bekannt und kommerziell zugänglich. Beispielsweise kann das System Vivostat® der Vivostat A/S, Dänemark, eingesetzt werden, dabei werden Fibrinoproteine als Vorläufer für Fibrinogen eingesetzt. Andere ähnliche Systeme sind bekannt, mit denen ebenfalls aus Patientenblut Fibrinpräparate gewonnen werden können, die üblicherweise bei bestimmten chirurgischen Operationstechniken zum Einsatz kommen (siehe beispielsweise EP 2 010 238 A2 oder EP 1 061 931 A2).

Die Erfindung ermöglicht daher die Herstellung eines artifiziellen Gewebekonstrukts aus Materialien, die einer einzigen Blutprobe entnommen werden können, und zwar in Form einer primär azellulären Proteinpräparation aus Blutplasma (patienteneigene Fibrinpräparation) und von zur Besiedlung geeigneten Zellen, die aus dem Monozytenfilm der gleichen Probe gewonnen und expandiert werden können. Mit diesen Materialien ist ein weitgehend autologes Konstrukt zu erhalten, das die Gefahr pathologischer Reaktionen bei Verwendung dieses Konstrukts im Körper eines Patienten mit guter Wahrscheinlichkeit ausschließen kann.

Das Verfahren ist vorzugsweise so ausgestaltet, dass die für die Besiedlung gewählten und expandierten Zellen in eine noch formbare, vorzugsweise flüssige, pastöse oder gelförmige Proteinzubereitung eingebracht und zu einem formstabilen Konstrukt weiterverarbeitet werden.

Dabei ist es bevorzugt, dass die Proteinzubereitung mit den Zellen auf eine Platte gegossen oder in eine Gussform eingebracht, fest werden gelassen und entformt wird. Die Gussform kann beispielsweise für ein einfaches Gefäßkonstrukt die Negativform für ein Zylinderrohr besitzen und die Zubereitung aus dem Matrixmaterial, nämlich der Proteinzubereitung und den Zellen, kann in diese Form in einem Spritzgussverfahren eingebracht werden. Nach der Verfestigung des Präparats durch erstes Anwachsen der Zellen kann das soweit gestaltete Gewebekonstrukt entformt und weiter verarbeitet werden.

In einem weiteren Schritt des Herstellungsverfahrens wird das formstabile Konstrukt nach dem Entformen aus der Gussform, soweit eine solche verwendet wurde, in einem Bioreaktor perfundiert und weiter kultiviert.

Das spezielle "Spritzgussverfahren" nach der Erfindung bietet besondere Vorteile. Auf sehr einfache und zügige Weise können ganz verschieden geformte Konstrukte erhalten werden. Anders als bei manchen bekannten Besiedlungsverfahren ist es hier möglich, verzweigte Gefäßprothesen zu erzeugen. Der Zeitaufwand für das Verfahren liegt insgesamt einschließlich Formen und Weiterbehandeln im Bioreaktor unter Perfusion bei ca. 2 bis 3 Wochen. Die Nachbehandlung in einem Bioreaktor ist besonders vorteilhaft, da sich herausgestellt hat, dass eine solche Behandlung unter naturähnlichen Bedingungen dem Gewebekonstrukt zusätzliche Festigkeit verleiht.

Ohne die Weiterbehandlung im Bioreaktor kann ein einfaches Gewebekonstrukt, beispielsweise ein Patch, auch in Nährlösung eingelegt und darin stabilisiert und aufbewahrt werden. Die Nährlösung sollte das Aufbewahrungsgefäß durchströmen, sie ist regelmäßig zu wechseln.

In Weiterbildung der Erfindung ist allerdings vorgesehen, das Konstrukt einer Perfusion im Bioreaktor zu unterziehen. Reaktoren, die für die verschiedenste Gewebekonstrukte geeignet sind, sind im Stand der Technik bekannt. Für röhrenförmige Konstrukte bietet sich beispielsweise ein Reaktor an, wie in der DE 199 15 610 A1 (Bader) dargestellt, oder ein solcher wie in EP 0 320 441 B1 (Sulzer) beschrieben. Das röhrenförmige Gefäß sollte in einem solchen Reaktor eingespannt und so von Medium oder Blut durchströmt werden, wie es der späteren natürlichen Einbausituation im Körper am ehesten entspricht. Besonders bevorzugt ist es, wenn das Durchströmen pulsatil erfolgt, um dem Einfluss des Herzschlags und des Blutkreislaufes nachzuahmen. Weiterhin ist es bevorzugt, wenn das Gefäß während des Durchströmens, sowohl bei gleichmäßigem Durchströmen als auch im Pulsbetrieb, zeitweise einem erhöhten Druck ausgesetzt wird. Alle diese Maßnahmen verbessern die mechanische Festigkeit des erhaltenen Konstrukts und stimulieren die Organisation der Zellen zu einem natürlichen Aufbau.

Das Prinzip des erfindungsgemäßen Besiedlungsverfahrens besteht unter anderem darin, dass zunächst eine sehr unstrukturierte "provisorische" Zellmatrix-Struktur generiert wird, die weder den hierarchischen strukturierten zellulären Aufbau noch einen vergleichbaren Aufbau der extrazellulären Matrix einer Gefäßwand zeigt. Erst die Kultivierung, vorzugsweise unter möglichst physiologischen Bedingungen in einem Bioreaktor mit Perfusion der Segmente, führt zum einen zur hierarchischen Anordnung der Zellen mit einer konfluenten Schicht von Endothelzellen, die das Lumen auskleiden, und dreidimensional angeordnete Muskelzellen in der Matrix. Zudem bauen die angesiedelten Zellen die Trägerstruktur um, indem das Fibrin abgebaut und ein Gerüst neuer Matrixproteine gebildet wird. Durch den Einfluss der EPC wird zudem die Vaskularisierung gefördert. Um diese Reifung der provisorischen Struktur zu einem Segment, das einem Gefäß ähnelt, zu erreichen, sind die beschriebenen Zelleigenschaften von entscheidender Bedeutung.

Die Erfindung umfasst weiterhin allgemein die Verwendung von aus Blut eines Patienten gewonnenen Early Outgrowth Endothelial Progenitor Cells gemeinsam mit Late Outgrowth Endothelial Progenitor Cells ohne vorheriges mehrfaches Passagieren in einem Besiedlungsverfahren für die Herstellung eines patientenspezifischen bioartifiziellen Gewebekonstrukts, insbesondere für ein Bypass-Material, prothetischen Gefäßersatz, Gewebepatches, Gewebeersatzstücke oder Conduits, gemäß den Ansprüchen.

Auch in anderen als den hier speziell geschilderten Besiedlungsverfahren ist die Verwendung der bei dieser Erfindung angegebenen speziellen EPC-Kultur bzw. -Fraktion sehr vorteilhaft. Eine sehr einfache Methode, um beispielsweise Gefäßprothesen aus Kunststoffmaterial mit Zellen zu besiedeln, besteht darin, ein Kunststoffröhrchen, beispielsweise aus PTFE, zu den Zellen in die Kulturflasche zu geben. Nach einigen Tagen haben sich bereits Endothelzellen auf der Prothese angesiedelt. Dabei ist die Überlebensrate dieser Endothelzellen deutlich höher als bei Verwendung mehrfach passagierter Zellen und das Anwachsen erfolgt gleichmäßiger. Nach diesem einfachen ersten Aufsiedeln der Zellen wird die Prothese in einem Bioreaktor perfundiert, wie oben bereits beschrieben. Es bildet sich eine konfluente Schicht von Endothelzellen aus. Diese Besiedlungstechnik ist mit reifen Endothelzellen, beispielsweise mit aus Aorten oder anderen Gefäßwänden gewonnenen isolierten Zellen, nicht möglich.

Eine weitere Besiedlungsmöglichkeit besteht darin, die Mischkulturen, beispielsweise auch in Suspension in der aus einer zweiten Menge Blut separierten Fibrinogenlösung, auf das Prothesenmaterial aufzusprühen.

Die Prothese bzw. das Kunststoffröhrchen, das auf einfache Weise besiedelt werden kann, kann zusätzlich mit einem Fibrinkleber beschichtet sein, der die Anhaftung der Zellen und die Ausbildung der konfluenten Schicht weiter befördert. Vorzugsweise werden bei allen derartigen Verfahren gemäß dieser allgemeinen Verwendung der EPC zusätzlich weitere Zelltypen eingesetzt, die vorzugsweise aus Blut oder Fettgewebe des Patienten, für den das Konstrukt gedacht ist, gewonnen werden, d.h. weitere autologe Zellen.

Weiterhin umfasst die Erfindung die Verwendung von aus Blut eines Patienten gewonnenen EOEC gemeinsam mit LOEC, ohne vorheriges mehrfaches Passagieren in einer Zusammensetzung für die Zelltransplantation. Gemäß dieser Verwendung kann die wie oben beschrieben erhaltene EPC-Zusammensetzung bei bekannten Zelltransplantationsverfahren eingesetzt werden, beispielsweise für Injektionen in ischämisches Gewebe, zur Rekonstruktion und Augmentation atrophierter oder chirurgisch entfernter Gewebe, für intra-myocordial-Injektionen oder sonstige Behandlungen mit körpereigenen Zellen im Körper eines Patienten selbst.

Schließlich richtet sich die Erfindung auf eine Zusammensetzung mit aus Blut eines Patienten gewonnenen, früh auswachsenden endothelialen Progenitorzellen (EOEC) gemeinsam mit spät auswachsenden endothelialen Progenitorzellen (LOEC), die ohne vorheriges mehrfaches Passagieren gewonnen werden, gemeinsam mit Zellen, ausgewählt aus der Gruppe Myoblasten, Myofibroblasten, glatte Muskelzellen, Vorläuferzellen der glatten Muskelzellen und Fibroblasten für die Verwendung in der Zelltransplantation. In einer Ausführungsform ist diese Zusammensetzung eine zur Injektion, z. B. in ischämisches Gewebe, intra-myocordial intraarteriell oder intravenös.

Im Folgenden wird die Erfindung anhand von Ausführungsbeispielen näher beschrieben:
Die Beispiele geben besonders vorteilhafte Ausführungsformen für die Erfindung wieder. Weitere Möglichkeiten ergeben sich aus der vorstehenden Beschreibung.

### Allgemeine Versuchsbeschreibung - Generierung aller verwendeten Zellen und einer Fibrinzubereitung aus Blut

### (1) Zellisolation und Kultivierung

Das Blut wird unter sterilen Kautelen entnommen und mit 100 Einheiten Heparin pro ml Blut vermischt. Zur Trennung der Zellen vom Plasma wird das Blut für 12 min. bei 600 g und 30 °C zentrifugiert. Das Plasma wird abpipettiert und zur Separierung einer Fibrinogenpräparation für mindestens 24 Stunden bei - 20 °C eingefroren. Nach Abnahme des Plasmas wird die Monozytenfraktion aspiriert und in gleichen Teilen in drei Gefäßen in Phosphate Buffered Saline Solution (PBS) resuspendiert. Zentrifugation der Suspensionen bei 300 g für 7 min. bei 4 °C. Abnahme des Überstandes und Resuspension des verbliebenen Zellpellets mit jeweils 10 ml Endothelzellmedium, Muskelzellmedium bzw. Fibroblastenmedium. Verwendet werden Endothelial Cell Growth Medium-2 (EGM-2), Smooth Muscle Growth Medium-2 (SGM-2) und Fibroblast Growth Medium- 2 (FGM-2) (jeweils von Lonza). Die Medien bestehen aus den jeweiligen Basalmedien, denen die jeweiligen Supplements beigefügt werden. Die Suspensionen werden in jeweils eine Zellkulturflasche gegeben und bei 37 °C und 5 vol.-% im Brutschrank inkubiert. Der erste Mediumwechsel erfolgt nach zwei Tagen und dann alle drei Tage. Mit der Monozytenfraktion werden Vorläuferzellen für Endothelzellen und glatte Muskelzellen und zirkulierende Fibroblasten (Fibrozyten) in Kultur eingebracht. Während der Inkubation mit dem jeweiligen spezifischen Kulturmedium kommt es im Mittel nach sieben Tagen zum Auswachsen Kolonie-bildender Zellen.

Nach Erreichen der Konfluenz werden die Zellen mit Trypsin abgelöst und eins zu drei passagiert, also von einer auf drei Kulturflaschen aufgeteilt.

Die Endothelzellenkultur im Medium EGM-2 wird nach der ersten Passage verwendet, die Muskelzellenkultur im Medium SGM-2 und die Fibroblastenkultur im Medium FGM-2 werden vier bis fünf Mal passagiert.

### Protokoll (Kurzfassung)

1. Abnahme von 100 ml Blut und Zugabe von 100 Einheiten Heparin/ml Blut.
2. Zentrifugation mit 600 g bei 30 °C für 12 min. ohne Bremse.
3. Abnahme des Plasmas, das bei -20 °C für mindestens 24 Stunden eingefroren wird.
4. Abnahme des Monozytenfilms, der zu gleichen Teilen in dreimal jeweils 100 ml PBS resuspendiert wird.
5. Zentrifugation der Zellsuspension mit 300 g bei 4 °C für 7 min.
6. Abnahme des Überstandes und Resuspension des Zellpellets in:
   - EGM-2 für die Isolation von Endothelzellen
   - SGM-2 für die Isolation von glatten Muskelzellen
   - FGM-2 für die Isolation von Fibroblasten
7. Gabe der Zellsuspensionen in jeweils eine 75 cm²-Kulturflasche und Inkubation im Brutschrank bei 37 °C und 5 % CO₂.
8. Die Kulturen werden jeweils mit dem entsprechenden anfänglich verwendeten Medium weiter kultiviert. Der erste Mediumwechsel erfolgt nach zwei Tagen dann alle drei Tage.
9. Nach Erlangung der Konfluenz werden die Zellen mit Trypsin abgelöst und 1:3 passagiert.
10. Für Besiedlungen werden Endothelzellen (EOEC und LOEC) der 1. Passage verwendet und Muskelzellen und Fibroblasten der 4. und 5 Passage.

### (2) Generierung einer Fibrinpräparation als primär azelluläre Proteinmatrix

1. Nach Zentrifugation des heparinsierten Blutes wird das Plasma abpipettiert und bei -20 °C für mindestens 24 Stunden eingefroren.
2. Auftauen des eingefrorenen Plasmas zunächst bei Raumtemperatur dann im Kühlschrank auf 4 °C.
3. Zentrifugation des aufgetauten Plasmas mit 450 g für 3 min. bei 4 °C mit Bremse.
4. Abnahme des Überstandes. Das verbliebene Pellet geht ohne weitere Zusätze bei 37 °C in Lösung.
5. Die Fibrinpräparation kann bis zur weiteren Verwendung bis zu 30 Tage bei - 20 °C eingefroren werden.

### (3) Generierung eines bioartifiziellen Gefäßersatzes

1. Fibrinpräparation aus (2) wird auf 37 °C erwärmt.
2. Generierung einer Thrombinpräpration, 1 ml bestehen aus:
   - 20 Einheiten bovines Thrombin
   - 400 µl Kalziumchloridlösung (50 mmol/L)
   - 300 µl Protamin (5000 Einheiten/ml)
   - 300 µl Aprotininlösung (230.000 KIU/ml)
3. Ablösen der Zellen aus (1) mit Trypsin, Zentrifugation mit 300 g für 7 min. und Resuspension der Zellen in der Thrombinpräparation mit einer Zelldichte 1,5 x 10⁶ Muskelzellen, 2 x 10⁵ Fibroblasten und 1 x 10⁵ Endothelzellen pro ml.
4. Gabe der Fibrinpräparation und der Thrombinpräparation im Verhältnis 1:1 unter simultaner Mischung über einen Y-Konnektor in eine Gussform (jeweils 4 ml für ein 15 cm langes Segment mit einem Innendurchmesser von 5 mm).
5. Nach 3 Minuten wird das Konstrukt aus der Gussform genommen und in eine Kulturflasche gegeben. Inkubation in EGM-2 mit Zusatz von 10.000 KIU Aprotinin im Brutschrank für eine Woche. Das Medium wird alle zwei Tage gewechselt.
6. Nach einer Woche Kultivierung unter statischen Bedingungen wird das Segment in einen Bioreaktor eingespannt. Der Bioreaktor selbst wird mit 50 ml SGM-2 plus 10.000 KIU Aprotinin pro ml befüllt. Das Segment wird mit EGM-2 perfundiert. Die Perfusion erfolgt zunächst mit 20 ml/min und einem Druck von 10 mmHg. Die Perfusionsrate wird dann täglich um 5 ml/min pro Tag gesteigert bis zu einer Rate von 50 ml/min, die dann weiter beibehalten wird. Danach wird der Druck um 10 mmHg pro Tag gesteigert bis zu einem Mitteldruck von 60 mmHg, der bis zum Ende beibehalten wird. Insgesamt erfolgt die Perfusion im Bioreaktor für drei Wochen. Das Medium wird einmal pro Woche gewechselt. Der Gasaustausch erfolgt über PTFE-Filter auf dem Reservoirgefäß des Perfusionskreislaufes.

### TESTERGEBNISSE

### 1. Adhäsionsvergleich

Aus Blut isolierte Endothelzellen (EOEC und LOEC) der ersten Passage wurden auf eine Testmatrix aus PTFE und eine Testmatrix aus mit Fibrinkleber bestrichenem PTFE aufgebracht. Zum Vergleich wurden aus Aorten gewonnene adulte Endothelzellen (aEC) auf die gleichen Testmatrizes bzw. Unterlagen aufgebracht.

Die Ansiedlung der erfindungsgemäßen EPC führte auf beiden Oberflächen zur Ausbildung konfluenter Schichten, die Ansiedlung von aEC auf beiden Oberflächen nicht.

Mittels eines Apoptose-Assays (in situ cell death detection kit, Roche) wurden in den Kulturen von aEC auf PTFE und Fibrinkleber Apoptosespezifische DNA-Fragmente nachgewiesen, während dies für die amplizierten EPCs nicht der Fall war.

Ergebnis: Das Aufbringen der erfindungsgemäßen EPC führt schnell zu konfluenten Schichten aus lebensfähigen Zellen. Die Ergebnisse sind positiv bis zu in Mittel 20 ± 7 Tage nach Isolation.

### 2. Versuche im Bioreaktor

Ein wie oben beschrieben erhaltenes Gefäßersatzstück aus der mit Zellen versetzten Fibrinpräparation mit einer Länge von 15 cm und einem Innendurchmesser von 5 mm (Volumen der Präparation ca. 4 ml), wurde in einem Bioreaktor des Typs, wie beschrieben, bei Williams C. and Wick T.M; "Perfusion Bioreactor for small diameter tissue-engineered arteries", Tissue Eng. 2004; 10:930-41, eingebracht. Die Behandlung erfolgte unter Bedingungen, die den natürlichen Blutfluss simulierten.

Zunächst wurden Präparate untersucht, die in der Fibrinogenpräparation das spezifische EOEC/LOEC-Gemisch und SPC (vorwiegend LOSMC) enthielten. Die Kultivierung des daraus gegossenen Konstrukts unter dynamischen Bedingungen führte zu einer dreidimensionalen Anordnung der inkorporierten Zellen mit einer konfluenten Schicht aus LOEC (anti-CD31-FITC) auf der Oberfläche, die eine mehrlagige Schicht von LOSMC (anti-α-Actin-Cy3) bedeckten, wie durch fluoreszenzmikroskopische Aufnahmen verifiziert werden konnte.

In weiteren Experimenten wurden zusätzlich Fibroblasten in die Fibrinpräparation eingebracht. Hierdurch ergab sich eine vermehrte Bildung von Kollagen in den bioartifiziellen Gewebekonstrukten. Durch die vermehrte Bildung von Kollagen durch die zusätzlich angesiedelten Fibroblasten waren die generierten Segmente sehr gut nahtfähig und nochmals stabiler und druckbelastbarer als die ohne Fibroblasten genierten Segmente.

### 3. Zelleigenschaften

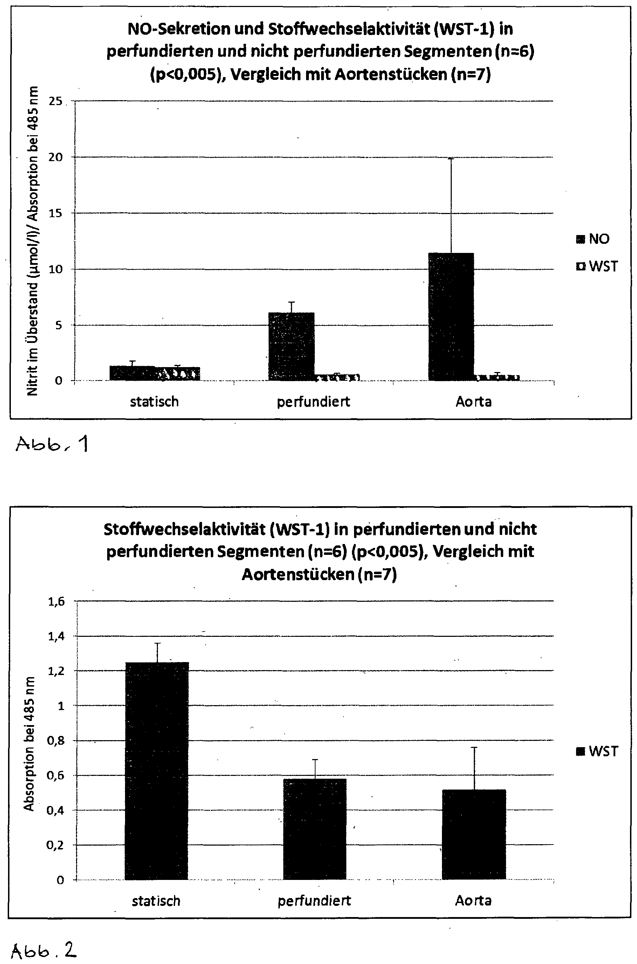

1. Es wurde die NO-Sekretion in perfundierten und nicht-perfundierten Segmenten verglichen. Hierfür wurde für sechs zuvor perfundierte Segmente der Nitritgehalt im Überstand in mmol/l UV-spektrometrisch bei 485 nm bestimmt. Die NO-Bildung ist in den perfundierten bioartifiziellen Segmenten vergleichbar mit denen von Aortenstücken (Mittelwert aus 7 Tests an Aortenstücken) und höher als bei in vergleichbarer Weise unter statischen Bedingungen kultivierten Segmenten (siehe Abbildung 1).
2. In entsprechender Weise wie bei 1. wurde die Stoffwechselaktivität an sechs perfundierten und nicht-perfundierten Segmenten sowie an sieben Aortenstücken getestet (p < 0,005). Die Stoffwechselselektivität wurde mit einem WST-1-Assay von Böhringer Mannheim getestet. Sie ist in perfundierten bioartifiziellen Segmenten vergleichbar mit denen von Aortenstücken, jedoch kleiner als bei den unter statischen Bedingungen kultivierten Segmenten (siehe Abbildung 2).

### Zusammenfassung

Das Verfahren zur Herstellung eines Gewebekonstrukts für medizinische Zwecke verwendet Endothelial Progenitor Cells (EPC), die nicht mehrfach passagiert wurden und einen Gehalt an EOEC (Early Outgrowth Endothelial Progenitor Cells), LOEC (Late Outgrowth Endothelial Progenitor Cells) und Muskelzellen und/oder Fibroblasten aufweisen. Diese Zellen und optional weitere werden als lebende Zellen auf eine Matrix aufgesiedelt oder in eine Matrix eingebracht, um nach weiteren Behandlungsschritten das Gewebekonstrukt zu ergeben. Die Matrix ist vorzugsweise eine Proteinzubereitung insbesondere eine Fibrinogenzubereitung. Sowohl die Zellen als auch die Fibrinogenzubereitung können aus einer einzigen Blutprobe eines Patienten gewonnen werden. Außer für die Herstellung von Bypass-Materialien, prothetischem Gefäßersatz, Gewebepatches, Conduits und dergleichen ist die EOEC-haltige EPC-Kultur oder -Suspension als Mittel für die Zelltransplantation geeignet.

## Patentansprüche

1. Verfahren zur Herstellung eines Gewebekonstrukts für medizinische Zwecke, bei welchem lebende Zellen in vitro auf eine Matrix aufgesiedelt oder in eine Matrix eingebracht werden, um nach weiteren Behandlungsschritten das Gewebekonstrukt zu ergeben, **dadurch gekennzeichnet, dass** die primär auf- oder eingebrachten Zellen endotheliale Progenitorzellen (Endothelial Progenitor Cells = EPC) enthalten, die nicht mehrfach passagiert wurden und einen Gehalt sowohl an früh auswachsenden endothelialen Progenitorzellen (Early Outgrowth Endothelial Progenitor Cells = EOEC) als auch an spät auswachsenden endothelialen Progenitorzellen (Late Outgrowth Endothelial Progenitor Cells = LOEC) aufweisen, und dass gleichzeitig oder sequentiell außer den endothelialen Progenitorzellen ein oder mehrere Zelltypen, ausgewählt aus der Gruppe Myoblasten, Myofibroblasten, glatte Muskelzellen, Vorläuferzellen der glatten Muskelzellen und/oder Fibroblasten, auf- oder eingebracht werden.

2. Verfahren nach Anspruch 1, **dadurch gekennzeichnet, dass** bestimmte oder alle Zelltypen, wenigstens jedoch die endothelialen Progenitorzellen, aus Blut gewonnen werden.

3. Verfahren nach Anspruch 1, **dadurch gekennzeichnet, dass** die bestimmten Zelltypen aus Fettgewebe gewonnen werden.

4. Verfahren nach einem der Ansprüche 1 bis 3, **dadurch gekennzeichnet, dass** die Zellen auf eine Prothese oder eine Matrix aus wenigstens einem Material der Gruppe: in vivo nicht abbaubares biokompatibles Material, in vivo abbaubares biodegradables synthetisches Material; in vivo abbaubares biodegradables biologisches Material, insbesondere eine primär zellfreie Proteinzubereitung, aufgesiedelt oder in eine solche Matrix eingebracht werden.

5. Verfahren nach einem der Ansprüche 1 bis 4, **dadurch gekennzeichnet, dass** die Zellen in eine noch formbare, vorzugsweise flüssige, pastöse oder gelförmige Proteinzubereitung eingebracht und zu einem formstabilen Konstrukt weiterverarbeitet werden.

6. Verfahren nach Anspruch 4 oder 5, **dadurch gekennzeichnet, dass** die Proteinzubereitung eine Fibrinogenzubereitung ist, die vorzugsweise aus Blut gewonnen wurde.

7. Verfahren nach Anspruch 5 oder 6, **dadurch gekennzeichnet, dass** die die Zellen enthaltende Proteinzubereitung auf eine Platte gegossen oder in eine Gussform eingebracht, fest werden gelassen und entformt wird.

8. Verfahren nach Anspruch 7, **dadurch gekennzeichnet, dass** das formstabile Konstrukt nach dem Entformen aus der Gussform in einem Bioreaktor perfundiert wird.

9. Verwendung von aus Blut eines Patienten gewonnenen früh auswachsenden endothelialen Progenitorzellen (EOEC) gemeinsam mit spät auswachsenden endothelialen Progenitorzellen (LOEC), die ohne mehrfaches Passagieren gewonnen werden, und gleichzeitig oder sequentiell von Myoblasten, Myofibroblasten, glatten Muskelzellen oder deren Vorläufern, und/oder Fibroblasten in einem Besiedlungsverfahren für die Herstellung eines patientenspezifischen bioartifiziellen Gewebekonstrukts in vitro.

10. Verwendung nach Anspruch 9, **dadurch gekennzeichnet, dass** das Gewebekonstrukt ein Bypass-Material, ein prothetischer Gefäßersatz, ein Gewebepatch, ein Gewebeersatzstück oder ein Conduit ist.

11. Verwendung nach Anspruch 9 oder 10, **dadurch gekennzeichnet, dass** zusätzlich weitere Zelltypen aus Blut oder Fettgewebe eingesetzt werden.

12. Verwendung nach Anspruch 11, **dadurch gekennzeichnet, dass** die zusätzlich eingesetzten Zellen autologe Zellen des Konstrukt-Empfängers sind.

13. Zusammensetzung mit aus Blut eines Patienten gewonnenen früh auswachsenden endothelialen Progenitorzellen (EOEC) gemeinsam mit spät auswachsenden endothelialen Progenitorzellen (LOEC), die ohne vorheriges mehrfaches Passagieren gewonnen werden, gemeinsam mit Zellen, ausgewählt aus der Gruppe Myoblasten, Myofibroblasten, glatte Muskelzellen, Vorläuferzellen der glatten Muskelzellen und Fibroblasten für die Verwendung in der Zelltransplantation.

14. Zusammensetzung zur Verwendung nach Anspruch 13, wobei diese eine Zusammensetzung zur Injektion ist.

15. Zusammensetzung zur Verwendung nach Anspruch 14, wobei diese zur Injektion in ischämisches Gewebe, intra-myocordial, intraarteriell oder intravenös.

## Claims

1. Method for preparing a tissue construct for medical purposes, in which living cells are, in vitro, seeded onto a matrix or introduced into a matrix in order to yield the tissue construct following further treatment steps, **characterized in that** the primarily applied or introduced cells contain endothelial progenitor cells (EPC) which have not been passaged multiple times and have a content of both early outgrowth endothelial progenitor cells (EOEC) and late outgrowth endothelial progenitor cells (LOEC), and that, besides the endothelial progenitor cells, one or more cell types selected from the group consisting of myoblasts, myofibroblasts, smooth muscle cells, smooth muscle progenitor cells and/or fibroblasts are applied or introduced simultaneously or sequentially.

2. Method according to Claim 1, **characterized in that** particular cell types or all the cell types, but at least the endothelial progenitor cells, are obtained from blood.

3. Method according to Claim 1, **characterized in that** the particular cell types are obtained from adipose tissue.

4. Method according to any of Claims 1 to 3, **characterized in that** the cells are seeded onto a prosthesis or a matrix composed of at least one material from the group: in vivo nondegradable biocompatible material, in vivo degradable biodegradable synthetic material; in vivo degradable biodegradable biological material, more particularly a primarily cell-free protein preparation, or are introduced into such a matrix.

5. Method according to any of Claims 1 to 4, **characterized in that** the cells are introduced into a protein preparation which is still shapeable, preferably liquid, pasty or gel-like, and are further processed to yield a dimensionally stable construct.

6. Method according to Claim 4 or 5, **characterized in that** the protein preparation is a fibrinogen preparation which was obtained preferably from blood.

7. Method according to Claim 5 or 6, **characterized in that** the protein preparation containing the cells is cast onto a plate or introduced into a mold, allowed to set, and demolded.

8. Method according to Claim 7, **characterized in that** the dimensionally stable construct, after demolding from the mold, is perfused in a bioreactor.

9. Use of early outgrowth endothelial progenitor cells (EOEC) together with late outgrowth endothelial progenitor cells (LOEC) which are obtained from blood from a patient and which are obtained without multiple passaging, and, simultaneously or sequentially, of myoblasts, myofibroblasts, smooth muscle cells or the progenitors thereof, and/or fibroblasts in a colonization method for the preparation of a patient-specific bioartificial tissue construct in vitro.

10. Use according to Claim 9, **characterized in that** the tissue construct is a bypass material, a prosthetic vascular graft, a tissue patch, a tissue replacement part or a conduit.

11. Use according to Claim 9 or 10, **characterized in that** further cell types from blood or adipose tissue are additionally used.

12. Use according to Claim 11, **characterized in that** the cells additionally used are autologous cells from the construct recipient.

13. Composition of early outgrowth endothelial progenitor cells (EOEC) together with late outgrowth endothelial progenitor cells (LOEC) which are obtained from blood from a patient and which are obtained without prior multiple passaging, together with cells selected from the group of myoblasts, myofibroblasts, smooth muscle cells, smooth muscle progenitor cells and fibroblasts for use in cell transplantation.

14. The composition for use according to claim 13 whereby said composition is a composition for injection.

15. Composition for use according to claim 14 whereby said composition for injection is a composition for injection into ischemic tissue, in an intramyocardial, intraarterial or intravenous manner.

## Revendications

1. Procédé de fabrication d'un produit de synthèse tissulaire à des fins médicales, selon lequel des cellules vivantes sont établies sur une matrice ou incorporées dans une matrice in vitro pour obtenir le produit de synthèse tissulaire après d'autres étapes de traitement, **caractérisé en ce que** les cellules établies ou incorporées primairement sont des cellules progénitrices endothéliales (Endothelial Progenitor Cells = EPC) qui n'ont pas été soumises à plusieurs passages et qui présentent une certaine teneur en cellules progénitrices endothéliales à excroissance prématurée (Early Outgrowth Endothelial Progenitor Cells = EOEC) et en cellules progénitrices endothéliales à excroissance tardive (Late Outgrowth Endothelial Progenitor Cells = LOEC), et **en ce que** simultanément ou séquentiellement, en plus des cellules progénitrices endothéliales, un ou plusieurs types de cellules, choisies dans le groupe constitué par les myoblastes, les myofibroblastes, les cellules musculaires lisses, les précurseurs des cellules musculaires lisses et/ou les fibroblastes, sont établies ou incorporées.

2. Procédé selon la revendication 1, **caractérisé en ce que** certains ou tous les types de cellules, au moins toutefois les cellules progénitrices endothéliales, sont obtenues à partir de sang.

3. Procédé selon la revendication 1, **caractérisé en ce que** les types de cellules déterminés sont obtenus à partir de tissus graisseux.

4. Procédé selon l'une quelconque des revendications 1 à 3, **caractérisé en ce que** les cellules sont établies sur une prothèse ou une matrice en au moins un matériau du groupe constitué par : un matériau biocompatible non dégradable in vivo, un matériau synthétique biodégradable in vivo, un matériau biologique biodégradable in vivo, notamment une préparation protéinique primairement sans cellules, ou incorporées dans une telle matrice.

5. Procédé selon l'une quelconque des revendications 1 à 4, **caractérisé en ce que** les cellules sont incorporées dans une préparation protéinique encore façonnable, de préférence liquide, pâteuse ou en gel, et transformées en un produit de synthèse de forme stable.

6. Procédé selon la revendication 4 ou 5, **caractérisé en ce que** la préparation protéinique est une préparation de fibrinogène, qui a de préférence été obtenue à partir de sang.

7. Procédé selon la revendication 5 ou 6, **caractérisé en ce que** la préparation protéinique contenant les cellules est versée sur une plaque ou introduite dans un moule, laissée durcir et démoulée.

8. Procédé selon la revendication 7, **caractérisé en ce que** le produit de synthèse de forme stable est perfusé dans un bioréacteur après le démoulage du moule.

9. Utilisation de cellules progénitrices endothéliales à excroissance prématurée (EOEC) obtenues à partir du sang d'un patient conjointement avec des cellules progénitrices endothéliales à excroissance tardive (LOEC), qui ont été obtenues sans être soumises à plusieurs passages, et simultanément ou séquentiellement de myoblastes, de myofibroblastes, de cellules musculaires lisses ou leurs précurseurs et/ou de fibroblastes dans un procédé de colonisation pour la fabrication d'un produit de synthèse bioartificiel spécifique à un patient in vitro.

10. Utilisation selon la revendication 9, **caractérisée en ce que** le produit de synthèse tissulaire est un matériau de pontage, une prothèse de remplacement vasculaire, un patch tissulaire, une pièce de remplacement tissulaire ou un conduit.

11. Utilisation selon la revendication 9 ou 10, **caractérisée en ce que** d'autres types de cellules issues du sang ou de tissus graisseux sont en outre utilisées.

12. Utilisation selon la revendication 11, **caractérisée en ce que** les cellules utilisées en outre sont des cellules autologues du destinataire du produit de synthèse.

13. Composition comprenant des cellules progénitrices endothéliales à excroissance prématurée (EOEC) obtenues à partir du sang d'un patient conjointement avec des cellules progénitrices endothéliales à excroissance tardive (LOEC), qui ont été obtenues sans être soumises au préalable à plusieurs passages, conjointement avec des cellules choisies dans le groupe constitué par les myoblastes, les myofibroblastes, les cellules musculaires lisses, les précurseurs des cellules musculaires lisses et les fibroblastes, destinée à une utilisation dans la transplantation de cellules.

14. Composition destinée à une utilisation selon la revendication 13, dans laquelle celle-ci est une composition pour injection.

15. Composition destinée à une utilisation selon la revendication 14, dans laquelle celle-ci est pour l'injection dans un tissu ischémique, par voie intra-myocardique, intra-artérielle ou intraveineuse.
